# EUROPÄISCHE PATENTSCHRIFT

(11) **EP 1 894 052 B1**
(45) Veröffentlichungstag und Bekanntmachung des Hinweises auf die Patenterteilung: **18.01.2012**
(21) Anmeldenummer: 06761958.5
(22) Anmeldetag: 01.06.2006
(51) Int. Cl.: G02B 21/32, G01N 1/28, B01L 3/00, G01N 1/04

(54) **VERFAHREN UND VORRICHTUNG ZUM LASERINDUZIERTEN TRANSPORTPROZESS VON OBJEKTEN**
METHOD AND DEVICE FOR LASER-INDUCED TRANSPORT PROCESS OF OBJECTS
PROCEDE ET DISPOSITIF DE PROCESSUS DE TRANSPORT D'OBJETS INDUIT PAR LASER

(30) Priorität: 08.06.2005 DE 102005026540
(43) Veröffentlichungstag der Anmeldung: 05.03.2008
(73) Patentinhaber: Carl Zeiss MicroImaging GmbH, 07745 Jena (DE)
(72) Erfinder: SÄGMÜLLER, Bernd, Dr., 69514 Laudenbach (DE); NIYAZ, Yilmaz, 86159 Augsburg (DE); STALTMEIER, Thomas, 82383 Hohenpeissenberg (DE)
(74) Vertreter: Banzer, Hans-Jörg
(86) Internationale Anmeldenummer: PCT/EP2006/005230
(87) Internationale Veröffentlichungsnummer: WO 2006/131260

(56) Entgegenhaltungen:
- WO-A-01/73398
- WO-A2-02/42824
- WO-A2-03/029817
- DE-A1- 19 804 800
- DE-A1-102004 041 941

## Beschreibung

Die vorliegende Erfindung betrifft ein Verfahren sowie eine Vorrichtung zur Handhabung von biologischen oder nichtbiologischen Objekten nach dem Oberbegriff der Ansprüche 1 bzw. 18. Die Objekte können beispielsweise Teil einer auf einem Träger angeordneten biologischen oder nichtbiologischen Masse sein, von welcher sie durch Laserbestrahlung abgetrennt werden, um durch einen anschließenden laserinduzierten Transportprozess zu einem Auffangmedium transportiert zu werden.

Für eine Vielzahl von biologischen Untersuchungen ist es erforderlich, einzelne Zellen oder Strukturen aus einem Zellverband, wie etwa einem Gewebe oder einem histologischen Gewebepräparat, zu lösen. Dies kann beispielsweise mit mechanischen Mikrowerkzeugen, z. B. Mikrokapillaren oder Mikronadeln, erfolgen. Eine derartige Vorgehensweise ist jedoch mühsam und es besteht eine Kontaminationsgefahr für die herausgelösten Objekte. Ferner ist ein solches Verfahren kaum zu automatisieren.

In der WO 97/29355 A der Anmelderin wurde daher ein neuartiges Verfahren zum Sortieren und zur Gewinnung von einzelnen biologischen Objekten, die auf einem planaren Träger angeordnet sind, vorgeschlagen. Hierbei wird ein ausgewähltes biologisches Objekt von der umgebenden weiteren biologischen Masse durch einen Laserstrahl abgetrennt, so dass das ausgewählte biologische Objekt von der übrigen biologischen Masse freipräpariert ist. Das somit freipräparierte biologische Objekt wird anschließend mit Hilfe eines Laserschusses bzw. Laserimpulses von dem Träger zu einer Auffangvorrichtung durch einen katapultierartigen Prozess transferiert. Zu diesem Zweck kann die Auffangvorrichtung beispielsweise ein Auffangsubstrat umfassen, an welchem das transferierte biologische Objekt anhaftet. Weiterhin ist es möglich, das biologische Objekt in einen Auffangbehälter, z. B. eine Vertiefung bzw. einen Well einer so genannten Mikrotiterplatte, zu transferieren. Vorzugsweise wird eine adhäsive Schicht verwendet, um die transferierten Objekte an dem Auffangsubstrat zu fixieren. Als Träger der zu transferierenden Objekte bzw. der biologischen Masse, aus welcher die Objekte herausgetrennt werden, wird vorzugsweise eine UV-absorbierende Polymerfolie verwendet. Es ist jedoch auch ein Transport von anderen Trägersubstraten, beispielsweise Glas, möglich.

Ein zu separierendes biologisches Objekt einer auf dem Träger aufgebrachten biologischen Masse wird somit zunächst anhand einer Abbildung der biologischen Masse ausgewählt, aus der biologischen Masse ausgeschnitten und anschließend durch einen laserinduzierten Transportprozess zu der Auffangvorrichtung transportiert. Unter "biologischen Objekten" werden im Rahmen der vorliegenden Anmeldung vor allem lebende oder fixierte biologische Zellen oder Zellbestandteile verstanden, die Bestandteil eines flüssigen oder festen biologischen Materials, wie beispielsweise eines Zellegewebes, eines Abstriches, einer Zellkultur oder Ähnlichem sein können.

Mit Hilfe des zuvor beschriebenen Verfahrens können bestimmte Objekte aus einer biologischen Masse gezielt herausgelöst oder ausgesondert werden. Die biologischen Objekte können nebeneinander auf einem festen planaren Träger aufgebracht sein, wobei der Vorgang des Herauslösens oder Aussonderns innerhalb kurzer Zeit und berührungslos durchgeführt werden kann. Die Überlebensfähigkeit und die Morphologie der biologischen Objekte bleibt je nach gewählter Vorgehensweise erhalten, d. h. die biologischen Objekte werden durch den Abtrennprozess und den laserinduzierten Transportprozess nicht geschädigt oder beeinträchtigt oder gezielt disintegriert.

Zur weiteren Untersuchung der auf diese Weise gewonnenen biologischen Objekte ist es erforderlich, sie von dem Auffangsubstrat abzulösen. Wenn als Auffanggefäß die Kappe eines Mikrozentrifugenröhrchens gewählt wurde, kann dies beispielsweise in einem nachgelagerten Zentrifugationsschritt bewerkstelligt werden. Nach Ablösung von dem Auffangsubstrat kann dann eine weitere Prozessierung, z. B. eine Rekultivation, erfolgen. Die biologischen Objekte kommen somit im Laufe der Prozessierung mit einer zusätzlichen bzw. intermediären Oberfläche in Berührung. Dies kann insbesondere dann problematisch sein, wenn die intermediären Oberflächen Adhäsivmaterialien, wie zum Beispiel Silikone, PCR-Öl, Plaste, Puffermedien usw., umfassen. Zum einen kann es zu Veränderungen und/oder Beschädigungen des biologischen Materials kommen. Zum anderen wird durch den erforderlichen Ablösevorgang der Durchsatz verringert.

Für das Ablösen von lebenden Zellen von einer Oberfläche sind in der Regel aggressive Lösungsmittel (z. B. Trypsin) erforderlich. Hierbei können Veränderungen oder Schädigungen der Zellen auftreten. Beispielsweise bei der Behandlung von Stammzellen kann es zu Spontandifferenzierungsprozessen kommen.

In der WO 02/42824 A2 wird ein Aufnahmeelement zur Aufnahme von Objekten beschrieben, welches im Zusammenhang mit einem laserinduzierten Transportprozess einsetzbar ist. Das Aufnahmeelement kann eine Aufnahmefläche beinhalten, auf welcher eine Haftflüssigkeit aufgebracht ist.

Der vorliegenden Erfindung liegt daher die Aufgabe zu Grunde, ein vereinfachtes und effektiveres Verfahren zur Handhabung von biologischen oder nichtbiologischen Objekten bereitzustellen, bei welchen insbesondere die gehandhabten Objekte mit einem hohen Durchsatz und einer hohen Zuverlässigkeit weiterprozessiert werden können. Gleichzeitig sollen Beschädigungen oder Veränderungen an den Objekten so gering wie möglich sein und der Kontakt mit intermediären Oberflächen vermieden werden.

Diese Aufgabe wird erfindungsgemäß durch ein Verfahren mit den Merkmalen des Anspruches 1 sowie durch eine Vorrichtung mit den Merkmalen des Anspruches 18 gelöst. Die abhängigen Ansprüche definieren bevorzugte und vorteilhafte Ausführungsformen der Erfindung.

Die vorliegende Erfindung wird nachfolgend vornehmlich anhand der Handhabung von biologischen Objekten beschrieben. Die Erfindung ist jedoch ebenso für nichtbiologische Objekte (z. B. anorganische Materie) anwendbar, wobei es sich z. B. um mikroskopisch kleine Objekte aus Glas, Silica, Kunststoff oder künstlich hergestellte Vesikel in einer biologischen Masse handeln kann. Ebenso können die erfindungsgemäß gehandhabten Objekte aus einer nichtbiologischen Masse, z. B. einer Polymermasse oder dergleichen, herausgelöst sein.

Bei dem erfindungsgemäßen Verfahren befindet sich ein Objekt auf einem Träger und wird durch einen laserinduzierten Transportprozess von dem Träger zu einem Auffangmedium transportiert. Das Objekt wurde vorzugsweise zuvor durch Laserbestrahlung aus einer auf dem Träger befindlichen Masse herausgeschnitten. Erfindungsgemäß befindet sich das Auffangmedium in einem flüssigen Zustand. Als Auffangmedium können somit unterschiedliche Flüssigkeiten zum Einsatz kommen, wobei diese verschiedene Viskositäten aufweisen können, solange eine Fließbewegung möglich ist. Die Fließbewegung kann beispielsweise durch ein Pumpen- oder Spritzensystem bewerkstelligt werden.

Nach dem laserinduzierten Transportprozess befindet sich das Objekt somit in bzw. auf dem flüssigen Auffangmedium. Dies bietet den Vorteil, dass das Objekt für eine weitere Prozessierung in vorteilhafter Weise zusammen mit dem Auffangmedium transportiert werden kann. Dies kann beispielsweise durch ein automatisches Flüssigkeitshandhabungssystem bzw. Liquid-Handling-System bewerkstelligt werden. Auf diese Weise kann der Durchsatz gegenüber der herkömmlichen Vorgehensweise erheblich vergrößert werden. Ein Kontakt des Objekts mit intermediären Oberflächen wird vermieden. Insbesondere wird das Auffangmedium vorzugsweise derart ausgewählt, dass es mit der weiteren Prozessierung kompatibel ist, so dass das Objekt sofort mit einer für die weitere Prozessierung relevanten Flüssigkeit in Berührung kommt. Durch ein geeignetes Fördern des Auffangmediums kann das Objekt direkt in einen zur weiteren Prozessierung vorgesehenen Zielbehälter, z. B. einen Rekultivationsbehälter, überführt werden.

Das erfindungsgemäße Verfahren umfasst vorteilhafterweise weiterhin ein Überführen des Objekts zusammen mit dem Auffangmedium zu einem Zielort, z. B. in einen Zielbehälter. Hierfür bestehen vielfältige vorteilhafte Möglichkeiten, welche gemeinsam oder in Kombination eingesetzt werden können und einen hohen Durchsatz gewährleisten. Beispielsweise kann das Auffangmedium in eine Fließbewegung versetzt oder aktiv gefördert werden. Hierbei können beispielsweise ein Pumpsystem oder akustische Oberflächenwellen auf einem speziellen Chip, Ultraschallbewegung, welche z. B. durch eine Piezovorrichtung bewirkt wird, oder auch eine laserinduzierte Volumenvergrößerung (d. h. Kavitätsblasen) zum Einsatz kommen. Weiterhin kann das Auffangmedium in Tropfenform in einen Zielbehälter abgegeben werden, wobei vorzugsweise weiteres Auffangmedium aus einem Reservoir nachgeführt wird, um gleichzeitig einen Spülvorgang zu bewirken und/oder den Zielbehälter mit Auffangmedium zu befüllen.

Das Auffangmedium wird während des laserinduzierten Transportprozesses an einer Auffangvorrichtung gehalten. Das Überführen des Objekts an den Zielort umfasst dann vorzugsweise auch ein Bewegen der Auffangvorrichtung relativ zu dem Zielort. Das Bewegen der Auffangvorrichtung erfolgt bevorzugt in automatisierter Weise, z. B. mittels eines entsprechend ausgestalteten Roboters.

Die erfindungsgemäße Vorrichtung zur Handhabung von Objekten ist vorzugsweise zur Durchführung des oben beschriebenen Verfahrens ausgestaltet und umfasst eine Auffangvorrichtung, zu welcher das Objekt durch den laserinduzierten Transportprozess transportiert wird, eine Haltevorrichtung zum Halten des Trägers und eine Laseranordnung zum Bewirken des laserinduzierten Transportprozesses. Die Auffangvorrichtung ist dazu ausgestaltet, ein Auffangmedium zu halten, welches sich in einem flüssigen Zustand befindet. Das Halten des Auffangmediums an der Auffangvorrichtung erfolgt vorzugsweise durch Adhäsionskraft und/oder durch auf der Oberflächenspannung des Auffangmediums beruhenden Kräften.

Vorzugsweise umfasst die Vorrichtung darüber hinaus Abbildungsmittel zum Erzeugen einer Abbildung des Trägers bzw. darauf befindlicher Objekte. Anhand der Abbildung des Trägers ist es möglich, Objekte zu selektieren, welche anschließend durch den laserinduzierten Transportprozess zu dem Auffangmedium transportiert werden. Dies kann manuell, rechnergestützt oder automatisch basierend auf einer Bildverarbeitung erfolgen. Die Abbildungsmittel sind beispielsweise als inverses oder aufrechtes Mikroskop ausgestaltet oder umfassen allgemein für eine Abbildung im mikroskopischen Maßstab geeignete Komponenten. Die Abbildungsmittel können darüber hinaus vorteilhaft bei einer Überwachung des Handhabungsprozesses zum Einsatz kommen.

Erfindungsgemäβ umfasst die Auffangvorrichtung eine Spitze, an welcher das Auffangmedium in Form eines Tropfens gehalten wird. Die Spitze umfasst vorteilhafterweise eine Öffnung zur Abgabe des Auffangmediums aus einem Reservoir. Um eine zum Auffangen des Objekts geeignete Flüssigkeitsoberfläche des Auffangmediums bereitzustellen, sind vorzugsweise Mittel vorgesehen, durch welche eine zusätzliche Oberfläche bereitgestellt wird, um das Auffangmedium zu halten, und die Formgebung der Flüssigkeitsoberfläche zum Auffangen des Objekts gezielt beeinflusst werden kann. Zu diesem Zweck ist vorzugsweise ein Einsatz in der Öffnung der Spitze vorgesehen. Somit kann erfindungsgemäß die dargebotene Oberfläche des Auffangmediums gezielt beeinflusst werden und in eine geeignete Form gebracht werden.

Darüber hinaus sind Überwachungsmittel vorgesehen, durch welche das Auffangmedium an der Auffangvorrichtung überwacht wird. Die Überwachungsmittel können optisch ausgestaltet sein und beispielsweise eine Lichtschranke oder eine Bildverarbeitungseinrichtung umfassen. Weiterhin können die Überwachungsmittel auch derart ausgestaltet sein, dass die Überwachung mittels Ultraschall erfolgt.

Die Erfindung wird nachfolgend anhand von bevorzugten Ausführungsbeispielen unter Bezugnahme auf die beigefügten Zeichnungen näher erläutert.
Fig. 1 veranschaulicht schematisch den Gesamtaufbau einer Vorrichtung zur Handhabung von Objekten gemäß einem Ausführungsbeispiel der Erfindung.
Fig. 2 veranschaulicht schematisch einen laserinduzierten Transportprozess von einem Träger zu einem flüssigen Auffangmedium.
Fig. 3 veranschaulicht das Auffangmedium mit darin enthaltenen Objekten.
Fig. 4 veranschaulicht ein Überführen des Auffangmediums mit den darin enthaltenen Objekten an einen Zielort.
Fig. 5A und 5B veranschaulichen jeweils ein Beispiel für eine Auffangvorrichtung, an welcher das flüssige Auffangmedium gehalten wird.
Fig. 6 veranschaulicht schematisch eine Überwachung des Auffangmediums.

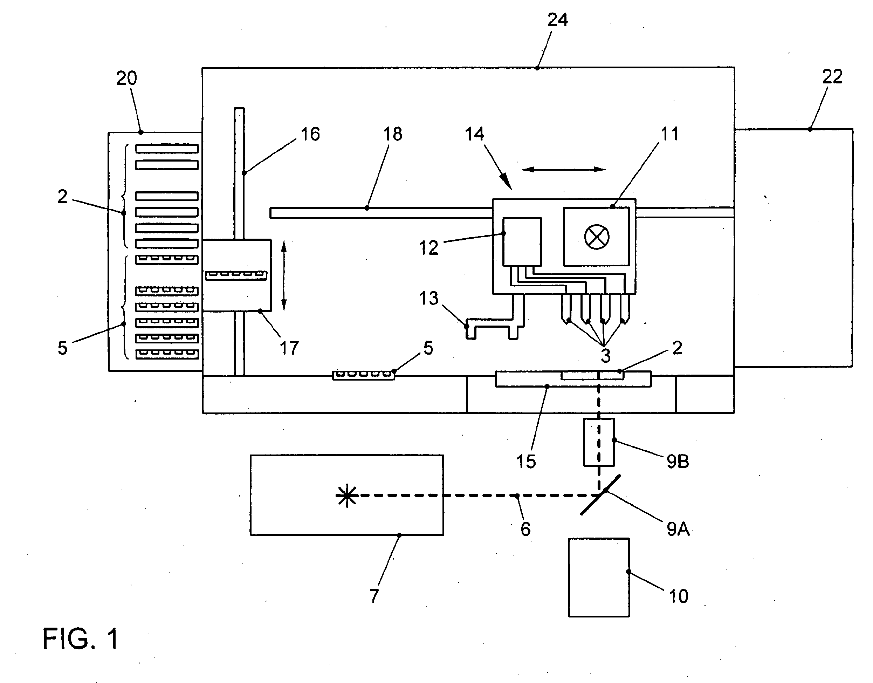

Fig. 1 zeigt den Gesamtaufbau einer Vorrichtung zur Handhabung von biologischen Objekten, welche mittels Laserbestrahlung aus einer auf einem Träger 2 befindlichen biologischen Masse herausgeschnitten werden. Die nachfolgenden Ausführungen lassen sich jedoch ohne weiteres auch auf die Handhabung von nichtbiologischen Objekten bzw. einer nichtbiologischen Masse übertragen. Die Vorrichtung ist modular aufgebaut und kann an unterschiedliche experimentelle Anforderungen individuell angepasst werden. Im vorliegenden Fall umfasst die Vorrichtung einen Mikroskopaufbau mit einer Beleuchtungseinheit 11 und einer Bildaufnahmeeinrichtung 10. Der Mikroskopaufbau wird in bekannter Weise dafür verwendet, eine Abbildung des Trägers 2 bzw. der darauf befindlichen Objekte zu erzeugen. Anstelle des dargestellten inversen Mikroskopaufbaus, bei welchem sich die Bildaufnahmeeinrichtung 10 unterhalb der Ebene des Trägers 2 befindet, wäre auch die Verwendung eines aufrechten Mikroskopaufbaus denkbar.

Ein wesentlicher Bestandteil der in Fig. 1 dargestellten Vorrichtung ist eine Laservorrichtung 7 zur Erzeugung eines Laserstrahls 6. Der Laserstrahl 6 wird über einen Spiegel 9A und eine Optik 9B in den Strahlengang des Mikroskopaufbaus eingekoppelt, so dass der Laserstrahl 6 auf die Ebene der Objekte auf dem Träger 2 fokussiert werden kann. Im vorliegenden Fall wird ein gepulster UV-Laser verwendet, dessen Wellenlänge z. B. 355 nm und dessen Impulsenergie z. B. 150 µJ beträgt. Die Impulsdauer beträgt 1 ns, während die Impulsfrequenz zwischen z. B. 1-200 Impulse pro Sekunde einstellbar ist. Die Laservorrichtung 7 kann beispielsweise mittels eines Stickstofflasers realisiert sein.

Die Laservorrichtung 7 emittiert einen Laserstrahl 6 mit einer festen Laserenergie. Der Laserstrahl 6 wird zum Zwecke einer so genannten Laser-Mikromanipulation und Laser-Mikrodissektion eingesetzt. Zu diesem Zweck wird der Laserstrahl 6 in Richtung eines motorisierten und computergesteuerten Mikroskoptisches 15 geführt, welcher als Haltevorrichtung für den Träger 2 dient. Der Mikroskoptisch 15 ermöglicht eine exakte Positionierung der auf dem Träger 2 befindlichen Objekte mit einer Präzision im NanometerBereich. Aufgrund der computergesteuerten Motorisierung des Mikroskoptisches 15 können laserbasierte Mikromanipulationsvorgänge automatisiert durchgeführt werden.

Der motorisierte Mikroskoptisch 15 ist entlang zweier linearer Achsen (x- und y-Richtung) verfahrbar. Die minimale Schrittgröße beträgt 20 nm, so dass auf dem Mikroskoptisch 15 befindliche Objekte mit sehr hoher Genauigkeit positioniert werden können. Die Genauigkeit und Reproduzierbarkeit des Verfahrvorgangs kann durch ein optisches Positioniersystem unterstützt bzw. gesteigert werden.

Weiterhin ist ein Robotik-Kopf 14 vorgesehen, welcher die Beleuchtungseinheit 11 für den Mikroskopaufbau trägt. Die Beleuchtungseinheit 11 umfasst vorzugsweise auch einen Kondensor und/oder einen Diffusor des Mikroskopaufbaus. Der Robotik-Kopf 14 kann weiterhin mit einer Feinpositionierungsvorrichtung versehen sein, welche z. B. auf Piezoaktuatoren basiert und eine Positionierung mit erhöhter Präzision im Nanometerbereich ermöglicht.

Der Robotik-Kopf 14 trägt Auffangvorrichtungen 3 zum Auffangen von Objekten, welche von dem Träger 2 durch einen laserinduzierten Transportprozess in Richtung des Robotik-Kopfes 14 katapultiert werden. Die Auffangvorrichtungen 3 sind an dem Robotik-Kopf 14 in verfahrbarer Weise angebracht, so dass sie ebenso wie der Mikroskoptisch 15 in der x- und y-Richtung positioniert werden können. Zusätzlich ist auch eine Verfahrbarkeit in der vertikalen Richtung bzw. der z-Richtung vorgesehen.

Der Robotik-Kopf 14 umfasst weiterhin eine Greifvorrichtung 13, mittels welcher der Träger 2 auf dem Mikroskoptisch 15 gegriffen werden kann. Die Greifvorrichtung 13 ist hierfür mit einer geeigneten vertikalen und horizontalen Beweglichkeit ausgestattet. Die Greifvorrichtung 13 ist somit zum Beladen und Entladen des Mikroskoptisches 15 mit dem Träger 2 geeignet. Darüber hinaus ist die Greifvorrichtung 13 auch dazu geeignet, einen Zielbehälter 5 zu greifen und in eine dafür vorgesehene Position zu befördern.

Sowohl die Träger 2 mit der darauf befindlichen biologischen Masse als auch die Ziel behälter 5 werden in einem Inkubator 20 aufbewahrt, welcher über eine Vielzahl von Aufnahmepositionen verfügt. Die Aufnahmepositionen des Inkubators 20 sind sowohl für die Zielbehälter 5 als auch für die Träger 2 geeignet. Dies ist dadurch bewerkstelligt, dass sowohl der Träger 2 als auch der Zielbehälter 5 in seinen Außenabmessungen einer Standard-Mikrotiterplatte entspricht bzw. in einer Aufnahmevorrichtung mit entsprechenden Abmessungen untergebracht ist. Der Inkubator 20 ist mit einer Be- und Entladevorrichtung 17 versehen, welche entlang einer Schiene 16 in der vertikalen Richtung verschiebbar ist und die Träger 2 und die Zielbehälter 5 automatisiert aus dem Inkubator 20 entnehmen bzw. in diesen einführen kann.

Bei der in Fig. 1 dargestellten Vorrichtung können somit sowohl die Träger 2 als auch die Zielbehälter 5 in automatisierter Weise ausgetauscht werden. Für die automatisierte Handhabung der Träger 2 und der Zielbehälter 5 ist der Robotik-Kopf 14 entlang einer Schiene 18 horizontal verfahrbar.

Weiterhin umfasst die Vorrichtung einen Kühlbehälter 22, in welchem wärmeempfindliche oder verderbliche Prozessmedien untergebracht sind. Insbesondere wird in dem Kühlbehälter 22 ein flüssiges Auffangmedium bzw. eine Auffangflüssigkeit aufbewahrt, welche zum Auffangen der von dem Träger 2 katapultierten Objekte verwendet wird. Zu diesem Zweck wird das flüssige Auffangmedium in die als Spritzen ausgestalteten Auffangvorrichtungen 3 aufgezogen und in Form eines Tropfens gegenüber dem Träger 2 positioniert. Durch den laserinduzierten Transportprozess wird ein Objekt von dem Träger 2 in bzw. auf den Tropfen des Auffangmediums an der Spitze der Auffangvorrichtung 3 transportiert. Anschließend wird die Spitze der Auffangvorrichtung 3 über dem Zielbehälter 5 positioniert und das Objekt durch eine gezielte tropfenförmige Abgabe des Auffangmediums von der Spitze der Auffangvorrichtung 3 in den Zielbehälter 5 überführt. Um die tropfenförmige Abgabe des Auffangmediums von der Spitze der Auffangvorrichtung 3 zu bewirken, ist der Robotik-Kopf 14 mit einer Steuer- und Betätigungsvorrichtung 12 für die als Spritzen ausgestalteten Auffangvorrichtungen 3 versehen. Mittels der Steuer- und Betätigungsvorrichtung 12 kann für jede der Auffangvorrichtungen 3 eine vorbestimmte Menge von Auffangmedium aus einer an ihrer Spitze befindlichen Öffnung abgegeben werden, so dass sich der Tropfen von der Auffangvorrichtung 3 löst und vorzugsweise weiteres Auffangmedium nachgeführt wird, um einen Spülvorgang zu bewirken. Selbstverständlich ist auch eine strahlförmige Abgabe des Auffangmediums möglich.

Die gesamte Anordnung aus Robotik-Kopf 14, Mikroskoptisch 15 und Zielbehälter 5 befindet sich unter einer so genannten Laminar-Flow-Box 24, in welcher aus Reinheitsgründen ein laminarer Luftstrom über die Komponenten der Vorrichtung geleitet wird. Vorzugsweise ist die Vorrichtung jedoch derart aufgebaut, dass der laminare Luftstrom im Bereich unterhalb des Robotik-Kopfes 14 abgelenkt wird, so dass der laserinduzierte Transportprozess durch den Luftstrom nicht beeinträchtigt wird.

Die Vorgehensweise zur automatisierten Handhabung von Objekten mittels des in Fig. 1 dargestellten Gesamtaufbaus soll nachfolgend näher erläutert werden.

Zunächst werden hierfür anhand einer mittels des Mikroskopaufbaus erzeugten Abbildung der biologischen Masse auf dem Träger 2 zu separierende Objekte ausgewählt. Dies geschieht vorzugsweise rechnergestützt wie in der WO 01/73398 A der Anmelderin vorgeschlagen, oder automatisch basierend auf einer elektronischen Bildverarbeitung. Falls erforderlich, werden die ausgewählten Objekte durch Laserbestrahlung von der Masse auf den Träger 2 abgetrennt, d. h. eine Mikrodissektion vorgenommen. Dies geschieht vorzugsweise, indem mittels des Mikroskoptisches 15 der Träger 2 relativ zu dem Laserstrahl 6 bewegt wird, so dass der Laserstrahl einen ausgewählten Bereich auf dem Träger umfährt, um ein darin enthaltenes Objekt freizupräparieren. Es kann auch eine geeignete Steuerung des Laserstrahls 6 vorgenommen werden, beispielsweise mittels eines Scanning-Systems mit einem so genannten Salvo- oder Prismenscanner. Anschließend erfolgt der laserinduzierte Transportprozess des Objekts von dem Träger 2 zu der Auffangvorrichtung 3. Zu diesem Zweck wird der Laserstrahl auf einen geeigneten Zielpunkt des Objekts gerichtet, und es wird ein Laserimpuls oder Laserschuss abgegeben, welcher das Objekt von dem Träger 2 zu der Auffangvorrichtung 3 transportiert. Da der Transport aufgrund der Bestrahlung mit dem Laserstrahl impulsartig oder katapultierartig erfolgt, d. h. es nach einer Beschleunigungsphase zu einem ballistischen Flug kommt, welcher im Wesentlichen nur durch das umgebende Medium (typischerweise Luft) beeinflusst ist, kann auch von einem Katapultieren des jeweils mit dem Laserstrahl bestrahlten Objekts gesprochen werden.

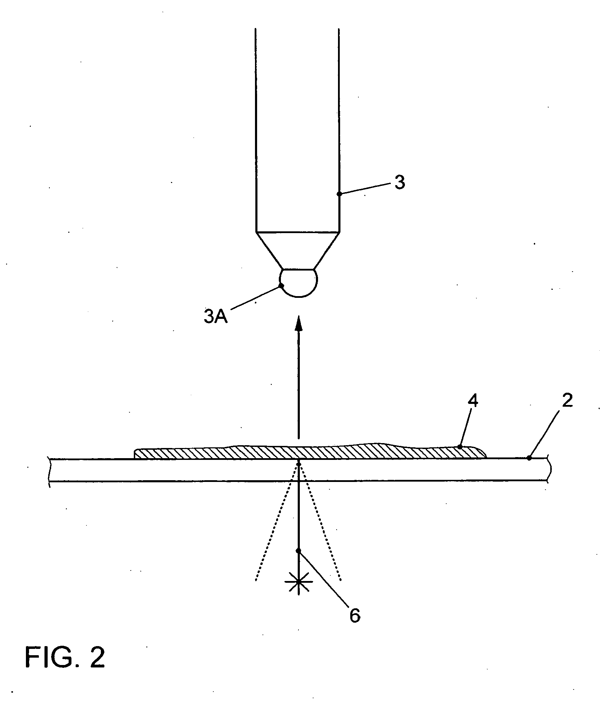

Fig. 2 zeigt schematisch die Anordnung von Träger 2, darauf.befindlicher biologischer Masse 4 und Auffangvorrichtung 3 während des laserinduzierten Transportprozesses. Wie bereits erwähnt, wird der Laserstrahl 6 auf einen geeigneten Zielpunkt gerichtet und ein Laserimpuls bzw. Laserschuss abgegeben. Hierdurch wird das ausgewählte Objekt von dem Träger 2 zu der Auffangvorrichtung 3 katapultiert, wie durch den Pfeil in Fig. 2 veranschaulicht. Die Auffangvorrichtung 3 ist als Spritze ausgestaltet, in welcher sich ein Reservoir mit Auffangmedium 3A befindet. An der Spitze der Auffangvorrichtung 3 befindet sich eine Öffnung, durch welche das Auffangmedium 3A abgegeben werden kann. Während des laserinduzierten Transportprozesses wird eine bestimmte Menge Auffangmedium 3A in Form eines Tropfens an der Spitze der Auffangvorrichtung 3 gehalten. Dieser Tropfen dient als Ziel für den laserinduzierten Transportprozess.

Durch automatisiertes Abarbeiten einer Liste von Positionen auf dem Träger 2 wird eine festgelegte Anzahl von Objekten aus der Masse 4 herausgeschnitten und in den Tropfen des Auffangmediums 3A katapultiert. Abhängig von der Oberflächenspannung des Auffangmediums 3A und von der Beschaffenheit der katapultierten Objekte kann auch ein Verbleib der Objekte an der Oberfläche des Tropfens erfolgen, wo sie durch Adhäsion anhaften.

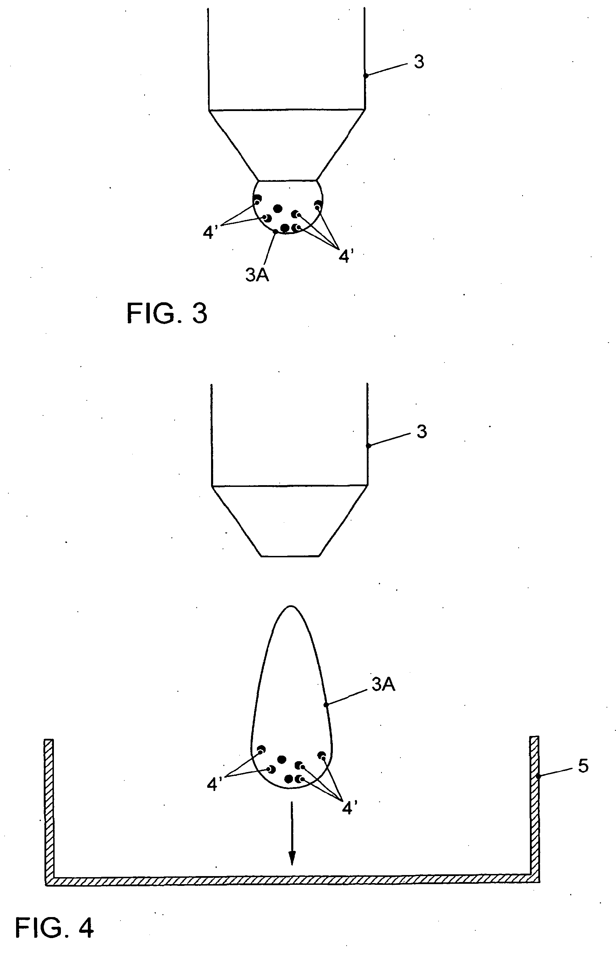

Fig. 3 zeigt den an der Spitze der Auffangvorrichtung 3 befindlichen Tropfen von Auffangmedium 3A mit darin befindlichen Objekten 4'. In diesem Zustand wird die Auffangvorrichtung 3 mittels des Robotik-Kopfes 14 zu dem Zielbehälter 5 bewegt.

Fig. 4 veranschaulicht die Abgabe der Objekte 4' in den Zielbehälter 5. Zu diesem Zweck wird gezielt eine vorbestimmte Menge von Auffangmedium aus der Auffangvorrichtung 3 abgegeben, so dass sich der Tropfen von Auffangmedium 3A von der Auffangvorrichtung 3 löst und in den Zielbehälter 5 fällt. Vorzugsweise wird darüber hinaus weiteres Auffangmedium 3A aus der Auffangvorrichtung 3 nachgeführt, um die Auffangvorrichtung 3 zu spülen und/oder um den Zielbehälter 5 für einen weiteren Prozessierungsschritt mit dem Auffangmedium 3A zu füllen. Letzteres ist insbesondere dann von Vorteil, wenn das Auffangmedium 3A mit dem nachfolgenden Prozessierungsschritt kompatibel ist bzw. eine für diesen Prozessierungsschritt bevorzugte Trägerlösung darstellt.

Nachfolgend können weitere Schritte zum Auswählen und Separieren von Objekten vorgenommen werden. Hierfür kann dieselbe Auffangvorrichtung 3 zum Einsatz kommen, oder die Aufnahmevorrichtung 3 kann ausgewechselt werden. Vorzugsweise enthält der Robotik-Kopf 14 mehrere Auffangvorrichtungen 3, welche beispielsweise mit unterschiedlichen Typen von Auffangmedium 3A gefüllt sein können, so dass sie für unterschiedliche nachfolgende Prozessierungsschritte geeignet sind.

Bei dem Zielbehälter 5 kann es sich beispielweise um eine so genannte Mikrotiterplatte mit sechs Vertiefungen bzw. Wells handeln, welche sich in vorteilhafterweise für eine Rekultivation eignet. Alternativ kann es sich auch um eine so genannte Petriperm-Schale handeln. Bei dem Auffangmedium 3A kann es sich beispielsweise um eine Denaturierungsflüssigkeit oder eine andere Prozessflüssigkeit, z. B. ein Medium für lebende Zellen, handeln.

Der oben beschriebene Prozess kann vorzugsweise zusätzlich ein weiteres Erzeugen von Abbildungen des Trägers 2 umfassen, um zu überwachen, ob der laserinduzierte Transportprozess erfolgreich war. Hierfür kann beispielsweise am Ende einer Serie von Mikrodissektionsvorgängen mit anschließendem laserinduzierten Transportprozess in der Abbildung des Trägers 2 überprüft werden, ob die ausgewählten Objekte erfolgreich entfernt wurden. Zusätzlich kann eine Aufnahme des Zielbehälters 5 erfolgen, um festzustellen, ob alle ausgewählten Objekte in den Zielbehälter 5 überführt wurden. Darüber hinaus erfolgt vorzugsweise eine optische Überwachung des Auffangmediums 3A an der Auffangvorrichtung 3. Auf diese Weise kann Dokumentationsvorschriften für den medizinischen Bereich, wie zum Beispiel 21CFR58.185 und 21CFR58.195 Rechnung getragen werden.

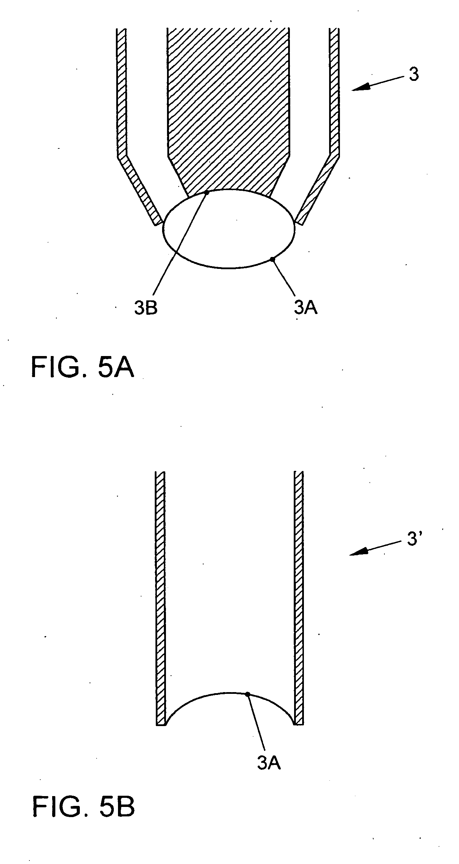

Das Auffangmedium 3A wird an der Auffangvorrichtung 3 durch Adhäsion und/oder Oberflächenspannung gehalten. Zu diesem Zweck ist die Auffangvorrichtung 3 mit einer entsprechenden Geometrie versehen. Zusätzlich kann das Halten des Auffangmediums 3A durch die Oberflächenbeschaffenheit der Auffangvorrichtung 3 bzw. ihrer Spitze beeinflusst werden. Mögliche Geometrien für die Spitze der Auffangvorrichtung 3 sind in Fig. 5A und Fig. 5B dargestellt.

Fig. 5A zeigt eine Spitze, welche zum Halten eines Tropfens von Auffangmedium 3A mit einer vergrößerten lateralen Ausdehnung ausgestaltet ist. Die vergrößerte laterale Ausdehnung des Tropfens bewirkt, dass die Zielgenauigkeit des laserinduzierten Transportprozesses weniger kritisch ist. Die Spitze der Auffangvorrichtung 3 weist eine allgemein zylindrische Form auf, welche sich in einem Endbereich nahe der Spitze konisch verjüngt. Die Spitze endet mit einer allgemein kreisförmigen Öffnung, durch welche das Auffangmedium 3A abgegeben werden kann und welche zur Aufnahme des Tropfens von Auffangmedium 3A dient. Im Inneren der Spitze befindet sich ein Kanal zur Zuführung des Auffangmediums 3A. Mittig in dem Kanal angeordnet ist ein Einsatz 3B, welcher eine im Bereich der Öffnung der Spitze befindliche Endfläche aufweist. Die Endfläche des Einsatzes 3B dient als zusätzliche Haltefläche für den Tropfen des Auffangmediums 3A. Weiterhin ist über die Positionierung der Endfläche des Einsatzes 3B und deren Formgebung auch die Formgebung des Tropfens von Auffangmedium 3A beeinflussbar.

Fig. 5B zeigt eine Spitze einer alternativen Auffangvorrichtung 3'. In diesem Fall ist eine im Wesentlichen zylinderförmige Ausgestaltung der Spitze vorgesehen, wobei die inneren Oberflächen der röhrenförmig ausgestalteten Spitze eine Oberflächenbeschaffenheit aufweisen, welche die Ausbildung eines nach innen gekrümmten Meniskus des Auffangmediums 3A in der Spitze bewirkt. Die Formgebung der Oberfläche des Auffangmediums kann durch entsprechende Maßnahmen beeinflusst werden. So bewirkt eine hohe Affinität der inneren Oberfläche der Spitze bezüglich des Auffangmediums 3A einen nach innen gekrümmten Meniskus, wie er in Fig. 5B dargestellt ist. Umgekehrt kann ein nach außen gekrümmter Meniskus durch eine verringerte Affinität der inneren Oberflächen der Spitze bezüglich des Auffangmediums 3A erreicht werden.

Selbstverständlich ist es auch möglich, den anhand von Fig. 5B beschriebenen Einsatz 3B mit einer gezielten Einstellung der Oberflächenaffinität zu kombinieren. Somit kann über die Geometrie oder die Oberflächenbeschaffenheit der Auffangvorrichtung 3 die Formgebung der dem Träger 2 dargebotenen Oberfläche des Auffangmediums 3 gezielt beeinflusst werden.

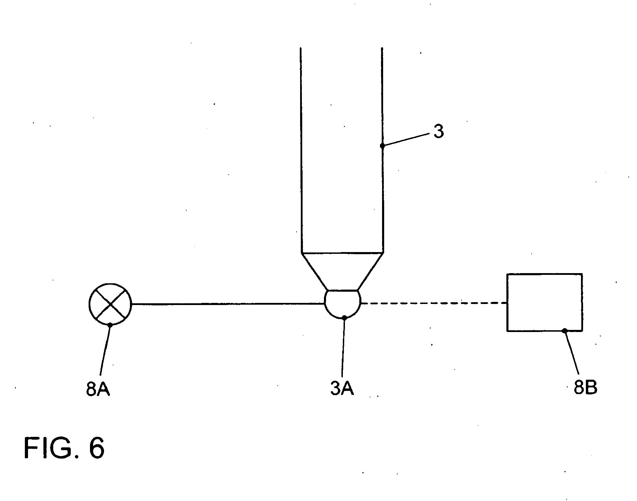

Fig. 6 veranschaulicht schematisch eine Überwachung des Auffangmediums 3A an der Auffangvorrichtung 3. Die in Fig. 6 schematisch dargestellten Überwachungsmittel umfassen eine Lichtschranke, welche eine Lichtquelle 8A und einen Sensor 8B aufweist. Der Tropfen des Auffangmediums 3A ist in dem Lichtweg positioniert, so dass sich die von dem Sensor 8B erfasste Signalstärke abhängig von der Beladung des Tropfens mit Objekten verändert. Weiterhin kann auf diese Weise auch überprüft werden, ob ein Tropfen von Auffangmedium 3A an der Spitze der Auffangvorrichtung 3 vorhanden ist. Letzteres kann eingesetzt werden, um sicherzustellen, dass vor dem laserinduzierten Transportprozess auch ausreichend Auffangmedium 3A an der Spitze der Auffangvorrichtung 3 vorhanden ist. Weiterhin kann auf diese Weise der anhand von Fig. 4 veranschaulichte Abgabevorgang des Auffangmediums 3A in den Zielbehälter 5 überwacht werden.

Alternative Ausgestaltungen der Überwachungsmittel können beispielsweise auf einer optischen Abbildung des Auffangmediums 3A mit einer automatischen Bildverarbeitung oder auf einem Ultraschall-basierten Überwachungsverfahren beruhen.

Weiterhin bestehen vielfältige Möglichkeiten, den Transport der separierten Objekte mittels des Auffangmediums 3A auszugestalten. So kann alternativ oder zusätzlich zu der anhand von Fig. 3 und 4 erläuterten tropfenförmigen Überführung des Auffangmediums 3A in den Zielbehälter 5 eine Vielzahl von weiteren Ansätzen verfolgt werden. Diese können beispielsweise ein Fördern des Auffangmediums 3A, ein Absaugen des Auffangmediums 3A oder eine anderweitig bewirkte Fließbewegung beinhalten. Weiterhin kann das Auffangmedium 3A auf speziellen Chips durch akustische Oberflächenwellen transportiert werden. So ist für bestimmte Anwendungen eine direkte Zuführung des Auffangmediums 3A mit den darin enthaltenen Objekten 4' auf einen Analysechip möglich und vorteilhaft.

Weiterhin ist das zuvor beschriebene Konzept eines Katapultierens auf oder in ein flüssiges Auffangmedium keinesfalls auf ein stationäres Auffangmedium, zum Beispiel in Form eines Tropfens, beschränkt. Beispielsweise ist es auch denkbar, einen kontinuierlichen Fluss von Auffangmedium vorzusehen, in welchen hinein das Objekt bzw. die Objekte katapultiert werden.

Die dargestellte Lösung zur Handhabung von mikrodissektierten biologischen Objekten bietet eine Vielzahl von Vorteilen. So gewährleistet sie einen geringen Materialverbrauch, eine leichte Automatisierbarkeit, die Möglichkeit eines hohen Durchsatzes, ein verringertes Risiko gegenüber einer Kontamination und geringere Lagerhaltungskosten für Verbrauchsmaterialien. Für eine Vielzahl von Präparations- und Analysevorgängen wird ein erheblich effektiverer Gesamtablauf ermöglicht. Auf eventuelle Autoklaviervorgänge von bei den herkömmlichen Verfahren verwendeten Verbrauchsprodukten kann verzichtet werden, da insbesondere keine eigens hierfür vorgesehenen Auffangbehälter oder Auffangsubstrate nötig sind. Durch eine präzise Flüssigkeitsführung wird eine hohe Genauigkeit und Zuverlässigkeit von Untersuchungen und Präparationen gewährleistet.

## Patentansprüche

1. Verfahren zur Handhabung von Objekten,
wobei sich ein Objekt (4') auf einem Träger (2) befindet und
wobei das Objekt (4') durch einen laserinduzierten Transportprozess von dem Träger (2) zu einem Auffangmedium (3A) transportiert wird,
wobei das Auffangmedium (3A) sich in einem flüssigen Zustand befindet und von einer Auffangvorrichtung (3) gehalten wird,
**dadurch gekennzeichnet,**
**dass** das Auffangmedium (3A) in Form eines Tropfens an einer Spitze der Auffangvorrichtung (3) gehalten wird.

2. Verfahren nach Anspruch 1,
**dadurch gekennzeichnet,**
**dass** nach dem laserinduzierten Transportprozess das Objekt (4') mit dem Auffangmedium (3A) zu einem Zielort (5) überführt wird.

3. Verfahren nach Anspruch 2,
**dadurch gekennzeichnet,**
**dass** das Überführen des Objekts (4') zu dem Zielort (5) ein Fördern des Auffangmediums (3A) umfasst.

4. Verfahren nach Anspruch 2 oder 3,
**dadurch gekennzeichnet,**
**dass** das Überführen des Objekts (4') zu dem Zielort (5) ein Bewirken einer Fließbewegung des Auffangmediums (3A) umfasst.

5. Verfahren nach einem der Ansprüche 2 bis 4,
**dadurch gekennzeichnet,**
**dass** das Überführen des Objekts (4') zu dem Zielort (5) eine tropfenförmige Abgabe des Auffangmediums (3A) umfasst.

6. Verfahren nach einem der Ansprüche 2 bis 5,
**dadurch gekennzeichnet,**
**dass** das Überführen des Objekts (4') zu dem Zielort ein Absaugen des Auffangmediums (3A) umfasst.

7. Verfahren nach einem der Ansprüche 2 bis 6,
**dadurch gekennzeichnet,**
**dass** das Überführen des Objekts (4') zu dem Zielort (5) ein Bewegen der Auffangvorrichtung (3) relativ zu dem Zielort (5) umfasst.

8. Verfahren nach einem der Ansprüche 2 bis 7,
**dadurch gekennzeichnet,**
**dass** das Überführen des Objekts (4') zu dem Zielort (5) ein Zuführen von weiterem Auffangmedium (3A) zu dem Auffangmedium (3A) mit dem Objekt (4') umfasst.

9. Verfahren nach einem der Ansprüche 2 bis 8,
**dadurch gekennzeichnet,**
**dass** das Überführen des Objekts (4') zu dem Zielort (5) automatisiert abläuft.

10. Verfahren nach einem der vorhergehenden Ansprüche,
**dadurch gekennzeichnet,**
**dass** das Auffangmedium (3A) durch Adhäsion und/oder Oberflächenspannung an der Auffangvorrichtung (3) gehalten wird.

11. Verfahren nach Anspruch 10,
**dadurch gekennzeichnet,**
**dass** die Formgebung einer dem Träger (2) dargebotenen Oberfläche des Auffangmediums (3A) durch die Geometrie der Auffangvorrichtung (3) gezielt beeinflusst wird.

12. Verfahren nach Anspruch 10 oder 11,
**dadurch gekennzeichnet,**
**dass** die Formgebung einer dem Träger (2) dargebotenen Oberfläche des Auffangmediums (3A) durch die Oberflächenbeschaffenheit der Auffangvorrichtung (3) gezielt beeinflusst wird.

13. Verfahren nach einem der vorhergehenden Ansprüche,
**dadurch gekennzeichnet,**
**dass** das Auffangmedium (3A) abhängig von einem nachfolgenden Prozessierungsschritt für das Objekt (4') ausgewählt wird.

14. Verfahren nach einem der vorhergehenden Ansprüche,
**dadurch gekennzeichnet,**
**dass** das Objekt (4') durch Laserbestrahlung von einer auf dem Träger (2) befindlichen Masse (4) abgetrennt wird.

15. Verfahren nach einem der vorhergehenden Ansprüche,
**gekennzeichnet durch**
ein Überprüfen des Ergebnisses des laserinduzierten Transportprozesses **durch** eine Überwachung des Auffangmediums (3A).

16. Verfahren nach Anspruch 15,
**dadurch gekennzeichnet,**
**dass** die Überwachung basierend auf einer optischen Signalverarbeitung erfolgt.

17. Verfahren nach Anspruch 15 oder 16,
**dadurch gekennzeichnet,**
**dass** das Überprüfen basierend auf akustischen Signalen im Ultraschallbereich erfolgt.

18. Vorrichtung zur Handhabung von Objekten,
wobei sich ein Objekt (4') auf einem Träger (2) befindet und
wobei das Objekt (4') durch einen laserinduzierten Transportprozess von dem Träger (2) zu einer Auffangrichtung (3) transportiert wird, umfassend:
die Auffangvorrichtung (3) zum Halten eines Auffangmediums (3A), welches sich in einem flüssigen Zustand befindet,
eine Haltevorrichtung (15) zum Halten des Trägers (2),
eine Laseranordnung (7, 9) zum Bewirken des laserinduzierten Transportprozesses,
**dadurch gekennzeichnet,**
**dass** die Auffangvorrichtung (3) eine Spitze umfasst, welche dazu ausgestaltet ist, das Auffangmedium (3A) in Form eines Tropfens zu halten.

19. Vorrichtung nach Anspruch 18,
**gekennzeichnet durch**
Abbildungsmittel (10, 11) zum Erzeugen einer Abbildung des Trägers (2).

20. Vorrichtung nach Anspruch 18 oder 19,
**dadurch gekennzeichnet,**
**dass** die Spitze eine Öffnung zur Abgabe des Auffangmediums (3A) umfasst.

21. Vorrichtung nach Anspruch 20,
**dadurch gekennzeichnet,**
**dass** in der Öffnung der Spitze ein Einsatz (3B) vorgesehen ist,
welcher eine zusätzliche Oberfläche bereitstellt, um das Auffangmedium (3A) zu halten.

22. Vorrichtung nach einem der Ansprüche 18 bis 21,
**dadurch gekennzeichnet,**
**dass** die Vorrichtung zur Durchführung des Verfahrens nach einem der Ansprüche 1 bis 17 ausgestaltet ist.

## Claims

1. A method of handling articles,
wherein an article (4') is present on a carrier (2) and
wherein the article (4') is conveyed from the carrier (2) to a collecting medium (3A) by a laser-induced conveying process,
wherein the collecting medium (3A) is in a liquid state and is held by a collecting apparatus (3),
**characterized in that** the collecting medium (3A) is held in the form of a drop at a tip of the collecting apparatus (3).

2. A method according to Claim 1, **characterized in that** the article (4') is transferred with the collecting medium (3A) to a destination (5) after the laser-induced conveying process.

3. A method according to Claim 2, **characterized in that** the transfer of the article (4') to the destination (5) includes a conveying of the collecting medium (3A).

4. A method according to Claim 2 or 3, **characterized in that** the transfer of the article (4') to the destination (5) includes effecting a flow movement of the collecting medium (3A).

5. A method according to any one of Claims 2 to 4, **characterized in that** the transfer of the article (4') to the destination (5) includes a discharge of the collecting medium (3A) in the form of a drop.

6. A method according to any one of Claims 2 to 5, **characterized in that** the transfer of the article (4') to the destination includes a sucking away of the collecting medium (3A).

7. A method according to any one of Claims 2 to 6, **characterized in that** the transfer of the article (4') to the destination (5) includes a movement of the collecting apparatus (3) relative to the destination (5).

8. A method according to any one of Claims 2 to 7, **characterized in that** the transfer of the article (4') to the destination (5) includes a supply of further collecting medium (3A) to the collecting medium (3A) with the article (4').

9. A method according to any one of Claims 2 to 8, **characterized in that** the transfer of the article (4') to the destination (5) takes place automatically.

10. A method according to any one of the preceding Claims, **characterized in that** the collecting medium (3A) is held on the collecting apparatus (3) by adhesion and/or surface tension.

11. A method according to Claim 10, **characterized in that** the shaping of a surface of the collecting medium (3A) presented to the carrier (2) is influenced in a purposeful manner by the geometry of the collecting apparatus (3).

12. A method according to Claim 10 or 11, **characterized in that** the shaping of a surface of the collecting medium (3A) presented to the carrier (2) is influenced in a purposeful manner by the surface characteristics of the collecting apparatus (3).

13. A method according to any one of the preceding Claims, **characterized in that** the collecting medium (3A) is selected in dependence upon a subsequent processing step for the article (4').

14. A method according to any one of the preceding Claims, **characterized in that** the article (4') is separated by laser radiation from a mass (4) present on the carrier (2).

15. A method according to any one of the preceding Claims, **characterized by** checking the result of the laser-induced conveying process by monitoring the collecting medium (3A).

16. A method according to Claim 15, **characterized in that** the monitoring is carried out on the basis of an optical signal processing.

17. A method according to Claim 15 or 16, **characterized in that** the checking is carried out on the basis of acoustic signals in the ultrasonic range.

18. An apparatus for handling articles,
wherein an article (4') is present on a carrier (2) and
wherein the article (4') is conveyed from the carrier (2) to a collecting apparatus (3) by a laser-induced conveying process, comprising:
the collecting apparatus (3) for holding a collecting medium (3A) which is in a liquid state,
a holding apparatus (15) for holding the carrier (2),
a laser arrangement (7, 9) for performing the laser-induced conveying process,
**characterized in that** the collecting apparatus (3) comprises a tip which is designed to hold the collecting medium (3A) in the form of a drop.

19. An apparatus according to Claim 18, **characterized by** imaging means (10, 11) for producing an image of the carrier (2).

20. An apparatus according to Claim 18 or 19, **characterized in that** the tip comprises an opening for the discharge of the collecting medium (3A).

21. An apparatus according to Claim 20, **characterized in that** an insert (3B), which provides an additional surface for holding the collecting medium (3A), is provided in the opening in the tip.

22. An apparatus according to any one of Claims 18 to 21, **characterized in that** the apparatus is designed to perform the method according to any one of Claims 1 to 17.

## Revendications

1. Procédé de manipulation d'objets,
dans lequel un objet (4') se trouve sur un support (2), et
dans lequel l'objet (4') est transporté du support (2) vers un milieu collecteur (3A) par un processus de transport induit par laser,
dans lequel le milieu collecteur (3A) se trouve à l'état liquide et est maintenu par un dispositif collecteur (3),
**caractérisé en ce que** le milieu collecteur (3A) est maintenu sous la forme d'une goutte sur une pointe du dispositif collecteur (3).

2. Procédé selon la revendication 1, **caractérisé en ce que** suite au processus de transport induit par laser, l'objet (4') avec le milieu collecteur (3A) est transféré vers un emplacement cible (5).

3. Procédé selon la revendication 2, **caractérisé en ce que** le transfert de l'objet (4') vers l'emplacement cible (5) comprend le fait de refouler le milieu collecteur (3A).

4. Procédé selon la revendication 2 ou 3, **caractérisé en ce que** le transfert de l'objet (4') vers l'emplacement cible (5) comprend le fait de provoquer un écoulement du milieu collecteur (3A).

5. Procédé selon l'une quelconque des revendications 2 à 4, **caractérisé en ce que** le transfert de l'objet (4') vers l'emplacement cible (5) comprend une distribution sous forme de goutte du milieu collecteur (3A).

6. Procédé selon l'une quelconque des revendications 2 à 5, **caractérisé en ce que** le transfert de l'objet (4') vers l'emplacement cible comprend une évacuation du milieu collecteur (3A).

7. Procédé selon l'une quelconque des revendications 2 à 6, **caractérisé en ce que** le transfert de l'objet (4') vers l'emplacement cible (5) comprend un déplacement du dispositif collecteur (3) par rapport à l'emplacement cible (5).

8. Procédé selon l'une quelconque des revendications 2 à 7, **caractérisé en ce que** le transfert de l'objet (4') vers l'emplacement cible (5) comprend le fait d'apporter un supplément de milieu collecteur (3A) au milieu collecteur (3A) avec l'objet (4').

9. Procédé selon l'une quelconque des revendications 2 à 8, **caractérisé en ce que** le transfert de l'objet (4') vers l'emplacement cible (5) se déroule automatiquement.

10. Procédé selon l'une quelconque des revendications précédentes, **caractérisé en ce que** le milieu collecteur (3A) est maintenu au niveau du dispositif collecteur (3) par adhérence et/ou tension superficielle.

11. Procédé selon la revendication 10, **caractérisé en ce que** la mise en forme d'une surface du milieu collecteur (3A), présentée au support (2), est affectée spécifiquement par la géométrie du dispositif collecteur (3).

12. Procédé selon la revendication 10 ou 11, **caractérisé en ce que** la mise en forme d'une surface du milieu collecteur (3A), présentée au support (2), est affectée par l'état de surface du dispositif collecteur (3).

13. Procédé selon l'une quelconque des revendications précédentes, **caractérisé en ce que** le milieu collecteur (3A) est sélectionné en fonction d'une étape de traitement consécutive, destinée à l'objet (4').

14. Procédé selon l'une quelconque des revendications précédentes, **caractérisé en ce que** l'objet (4') est séparé par rayonnement laser d'une masse (4) se trouvant sur le support (2).

15. Procédé selon l'une quelconque des revendications précédentes, **caractérisé par** un contrôle du résultat du processus de transport induit par laser au moyen d'une surveillance du milieu collecteur (3A).

16. Procédé selon la revendication 15, **caractérisé en ce que** la surveillance s'effectue sur la base d'un traitement de signal optique.

17. Procédé selon la revendication 15 ou 16, **caractérisé en ce que** le contrôle s'effectue sur la base de signaux acoustiques dans le domaine des ultrasons.

18. Dispositif de manipulation d'objets,
dans lequel un objet (4') se trouve sur un support (2), et
dans lequel l'objet (4') est transporté par un processus de transport induit par laser du support (2) vers un dispositif collecteur (3), comprenant :
le dispositif collecteur (3) pour maintenir un milieu collecteur (3A) qui se trouve à l'état liquide,
un dispositif de maintien (15) pour maintenir le support (2),
un ensemble laser (7, 9) pour provoquer le processus de transport induit par laser,
**caractérisé en ce que** le dispositif collecteur (3) comprend une pointe qui est équipée pour maintenir le milieu collecteur (3A) sous forme de goutte.

19. Dispositif selon la revendication 18, **caractérisé par** des moyens de formation d'image (10, 11) pour produire une image du support (2).

20. Dispositif selon la revendication 18 ou 19, **caractérisé en ce que** la pointe comprend une ouverture pour distribuer le milieu collecteur (3A).

21. Dispositif selon la revendication 20, **caractérisé en ce que** dans l'ouverture de la pointe, un insert (3B) est prévu qui fournit une surface supplémentaire pour maintenir le milieu collecteur (3A).

22. Dispositif selon l'une quelconque des revendications 18 à 21, **caractérisé en ce que** le dispositif est équipé pour réaliser le procédé selon l'une quelconque des revendications 1 à 17.
